# EUROPEAN PATENT SPECIFICATION

(11) **EP 0 815 474 B1**
(45) Date of publication and mention of the grant of the patent: **27.09.2000**
(21) Application number: 96908102.5
(22) Date of filing: 19.03.1996
(51) Int. Cl.: G01V 3/38, G01N 33/24

(54) **DETERMINING A PARAMETER IN A PHYSICAL SYSTEM**
FESTSTELLUNG EINES PARAMETERS IN EINEM PHYSISCHEN SYSTEM
DETERMINATION D'UN PARAMETRE DANS UN SYSTEME PHYSIQUE

(30) Priority: 20.03.1995 EP 95200674
(43) Date of publication of application: 07.01.1998
(73) Proprietor: SHELL INTERNATIONALE RESEARCH MAATSCHAPPIJ B.V., 2596 HR Den Haag (NL)
(72) Inventor: KOELMAN, Johannes, Maria, Vianney, Antonius, NL-2288 GD Rijswijk (NL); DE KUIJPER, Andre, NL-2288 GD Rijswijk (NL); SANDOR, Robert, Karl, Josef, NL-2596 HR The Hague (NL)
(74) Representative: Zeestraten, Albertus Wilhelmus Joannes
(86) International application number: PCT/EP96/01235
(87) International publication number: WO 96/29617

(56) References cited:
- US-A- 4 338 664
- US-A- 4 398 151
- GEOPHYSICS, vol. 59, no. 3, March 1994, TULSA US, pages 336-344, XP002007702 ALVARO BUENO BUORO, ET AL.: "Ambiguity analysis of well-log data"
- GEOPHYSICS, vol. 48, no. 9, September 1983, TULSA US, pages 1258-1268, XP002007703 A.E. BUSSIAN: "Electrical conductance in a porous medium"
- JOURNAL OF APPLIED PHYSICS, vol. 58, no. 8, October 1985, NEW YORK US, pages 3236-3243, XP002007704 SHECHAO FENG, ET AL.: "Geometrical model of conductive and dielectric properties of partially saturated rocks"

## Description

The present invention relates to a method of determining a parameter in a physical system representing the electrical behaviour of a composition. The method can for example be applied in well-logging whereby the objective is to determine a quantity of a fluid, such as water, brine or hydrocarbon, in an earth formation in order to assess whether such fluid can be exploited economically. In the art of well-logging, physical models are generally applied to represent the electrical behaviour of the earth formation. From the well-log results and the physical model, the content of a component of the earth formation is then determined.

A known method of determining parameters of such physical model is disclosed in "Electrical conductivities in oil-bearing shaly sands", Waxman M.H., and Smits L.J.M., SPE paper 1863-A presented at the 42nd Annual Fall Meeting, Houston, October 1-4, 1967. This paper discloses a method of determining a parameter of a physical model representing the electrical behaviour of an earth formation, consisting of defining said model by a relationship between the electrical conductivity of the formation, a plurality of physical variables of the formation and said parameter, selecting a sample which is representative for said formation and measuring the electrical conductivity of the sample for various magnitudes of the physical variables, and determining said parameter by applying the selected relationship to the measured conductivities of the sample.

In this known method the model, which is generally referred to as the Waxman-Smits model, is defined by the relationship: 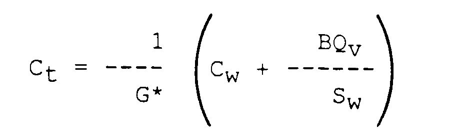 wherein;
- Cₜ =: the conductivity of the partially brine saturated formation represented by the sample
- C_{w} =: the conductivity of brine present in the formation;
- S_{w} =: the water saturation in the pore space (0..1), which equals 1-Sₒ where Sₒ denotes the hydrocarbon saturation;
- B =: the equivalent conductance of sodium clay-exchange cations as a function of C_{w} and temperature;
- Qᵥ =: the cation exchange capacity per unit pore volume;
- G* =: a formation factor of the formation represented by the sample;
- G*: is represented as${\text{G* = φ}}^{\text{-m*}} {\text{S}}_{\text{w}}^{\text{-n*}}$ wherein
φ = the pore space in the formation;
m* = a parameter to be determined, in the form of the cementation exponent;
n* = a parameter to be determined, in the form of the saturation exponent.

The parameters m* and n* characterise the response of the conductivity of the earth formation to changes in physical variables such as φ and S_{w}. In the known method these parameters are determined in a mutually independent way, namely:
- by using conductivity measurements in a non-hydrocarbon bearing zone in the earth formation or with laboratory conductivity measurements on a plurality of fully brine saturated samples; m* can be determined from the relation between log(C$\frac{\text{-1}}{\text{t}}$) and log(φ) or log(C_{w}·C$\frac{\text{-1}}{\text{t}}$) and log(φ), respectively; and
- by using laboratory conductivity measurements on a plurality of partially brine saturated samples, n* can be determined from the relation between

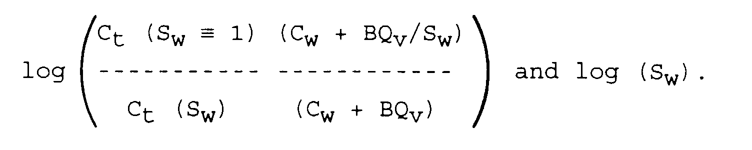

The results achieved with this known method are not always sufficiently accurate, probably because the parameters are determined in a non-optimal manner.

US-A-4,398,151 discloses a method of providing a corrected electrical log of shaly sand formations penetrated by a wellbore using in-situ measurements in the wellbore to determine cation exchange capacity per unit pore volume.

US-A-4,338,664 discloses a method of interpreting logging measurements made in a borehole for the evaluation of an earth formation. In this method a number of output parameters is determined from a number of logging measurements taken in the borehole by solving a plurality of response equations, wherein each response equation relates a single measured parameter to the output parameters. An incoherence function is derived from deviations between calculated values of the measured parameters and the actually measured values, whereby the deviations are multiplied by weighting factors.

It is an objective of the invention to provide an improved method of determining a parameter of a physical model representing the electrical behaviour of a composition.

The method according to the invention thereto comprises:
- defining said model by a relationship between at least one electrical property of the composition, a plurality of physical variables of the composition and said parameter;
- measuring a plurality of electrical properties including a first electrical property of the composition;
- selecting an incoherence function defining a difference between the measured electrical properties and the electrical properties as determined from said relationship, said incoherence function being such that the measured values are weighted in dependence of their accuracy; and
- determining said parameter by minimisation of said incoherence function, characterized in that said plurality of electrical properties includes a second electrical property of a sample which is representative for said composition, and that the method further comprises selecting the sample and measuring the second electrical property of the sample for various magnitudes of at least one of said physical variables.

By minimising the incoherence function each parameter is determined in a manner that all experimental data, both from in-situ measurements of the composition and from laboratory measurements on the sample representative for the composition, are taken into account in a weighted manner, i.e. in dependence of their respective accuracies. For example, in case the composition forms an earth formation, less accurate well-log measurements are taken into account in the incoherence function with less weight than accurate sample measurements. It is thereby achieved that the parameters are determined with increased accuracy resulting in a physical model, and applications thereof, of increased accuracy.

Suitably the step of determining said parameter by minimisation of the incoherence function includes an iterative process.

Advantageously a plurality of said parameters are determined simultaneously in minimising said incoherence function, each parameter being represented in said relationship.

The incoherence function can suitably be selected such that independent measurements of substantially equal accuracy are substantially equally represented therein.

Furthermore, the incoherence function is preferably monotonically increasing with the deviation between the measured electrical properties and the calculated electrical properties.

For example, the incoherence function can be of the form: 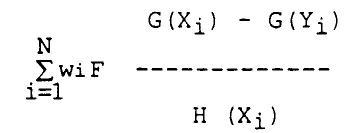 wherein
i=1..N, N being the number of physical measurements,
Xᵢ represents the measured electrical property,
Yᵢ represents the calculated electrical property,
G and H represent functions of the electrical property (H(Xᵢ)>0)
F represents a function with ∀*X*≠0[*F*(*X*)>*F*(0)],
wᵢ represents the weight attributed to the physical measurement (wᵢ≥0).

The functions F, G and H can be selected as follows:${\text{F(X) = |X|}}^{\text{α}} \text{,α ∈ R, α > 0}$${\text{G(X) = X}}^{\text{β}} \text{,β ∈ R, β ≠ 0}$$\text{H(X) = 1 or H(X)= G (X).}$

Another suitable form of the incoherence function is: 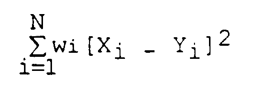 wherein
i=1..N, N being the number of physical measurements,
Xᵢ represents the measured electrical property,
Yᵢ represents the calculated electrical property,
wᵢ represents the weight attributed to the physical measurement (wᵢ≥0).

Furthermore, the incoherence function can be of the form: 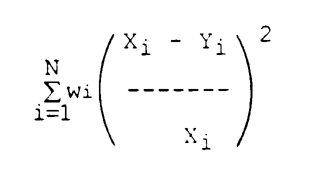 wherein
i=1..N, N being the number of physical measurements,
Xᵢ represents the measured electrical property,
Yᵢ represents the calculated electrical property,
wᵢ represents the weight attributed to the physical measurement (wᵢ≥0).

In a practical embodiment of the invention said at least one electrical property of the composition as defined in said relationship and the measured second electrical property of the sample are selected from the conductivity, the resistivity and the membrane potential.

Furthermore, it is suitable to select the measured first electrical property of the composition from the conductivity and the resistivity.

The composition can include, for example, a fluid and the model then forms a saturation model for the fluid in the composition.

When applying the invention in the technique of wellbore logging, said composition comprises an earth formation, and the model forms a saturation model for a selected one of brine and hydrocarbon fluid contained in the formation.

In selecting said relationship it is preferred to include therein the electrical conductivity of the composition and a plurality of composition parameters including, for each component in the composition, physical parameters representing the electrical conductivity and the volume fraction of the component, said relationship being such that the components are substantially equally represented in said relationship by means of said physical parameters.

The model used in applying the invention can be, for example, the Waxman-Smits model, the Archie model, the Poupon-Leveaux model, the Simandoux model, the Dual-Water model of Clavier-Coates-Dumanoir, or the effective medium model of Spalburg.

Suitably said relationship is selected to include the electrical conductivity of the composition and a plurality of composition parameters including, for each component in the composition, physical parameters representing the electrical conductivity and the volume fraction of the component, said relationship being such that the components are substantially equally represented in said relationship by means of said physical parameters. It is to be understood that each one of the components is represented in the relationship in substantially the same manner as any other of the components.

For example, the relationship can be selected as:${\text{(σ}}_{\text{eff}} {\text{- σ}}_{\text{0}} {\text{).(Lσ}}_{\text{eff}} {\text{+ (1-L)σ}}_{\text{0}} {\text{)}}^{\text{-1}} {\text{= Σ φ}}_{\text{k}} {\text{(σ}}_{\text{k}} {\text{- σ}}_{\text{0}} {\text{).(Lσ}}_{\text{k}} {\text{+ (1-L)σ}}_{\text{0}} {\text{)}}^{\text{-1}}$ wherein
σ₀ represents an auxiliary parameter in the form of a conductivity tensor
k = 1 ... N, N being the number components in the composition
σ_{eff} represents the conductivity tensor of the sample
σₖ represents the conductivity tensor of component k
φₖ represents the volume fraction of component k
L represents a depolarisation tensor.

Advantageously said auxiliary parameter is selected to be:${\text{σ}}_{\text{0}} {\text{= Σ h}}_{\text{k}} {\text{σ}}_{\text{k}}$ wherein hₖ represents a mixing coefficient tensor pertaining to component k.

Preferably each mixing coefficient is selected as${\text{h}}_{\text{k}} {\text{= λ}}_{\text{k}} {\text{φ}}_{\text{k}} {\text{}}^{\text{ν}} {\text{(Σλ}}_{\text{n}} {\text{φ}}_{\text{n}} {\text{}}^{\text{ν}} {\text{)}}^{\text{-1}}$ wherein
k,n = 1 ... N, N being the number components in said plurality of components
λₖ represents a percolation rate tensor pertaining to component k
φₖ represents the volume fraction of component k
ν_{k,n} represents a percolation exponent pertaining to component k, n.

The invention will be described hereinafter by way of example in more detail.

Consider a set of measurements made on an earth formation which essentially consists of rock, brine, clay, and hydrocarbons. The measurements include:
A) logging data on a water-bearing interval in the formation, the data consisting essentially of conductivity and porosity measurements under known C_{w}, S_{w}(=1), and T conditions;
B) logging data on an interval in said formation susceptible of being hydrocarbon-bearing, which data essentially consist of conductivity and porosity measurements under known C_{w}, and T condition; and
C) laboratory data on core plugs representative for said formation, the data consisting essentially of conductivity, porosity, and Qᵥ measurements under controlled C_{w}, S_{w}, and T conditions.

Then, from the laboratory data, a standard correlation between the porosity and Qᵥ can be made, providing Qᵥ information along with the logging data. As saturation model the Waxman-Smits model referred to in the description of the prior art method, will be used, i.e.: 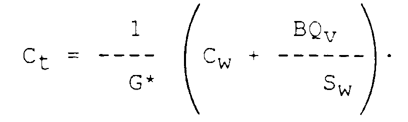 For the data mentioned under points A) and C) an incoherence function P(m*,n*) is defined as follows: 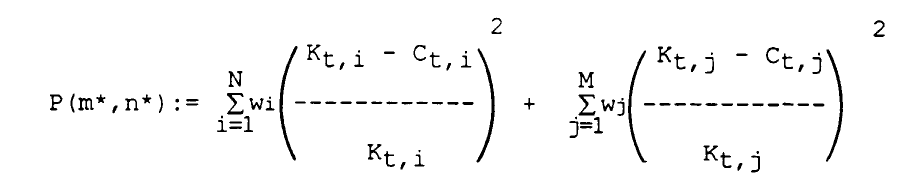 wherein
- i=1..N, N =: the total number of independent measurements referred to under A);
- wᵢ =: the weight attributed to the i-th measured conductivity of the rock;
- C_{t,i} =: the i-th calculated conductivity of the rock whose value depends on m* and n*;
- K_{t,i} =: the i-th measured conductivity of the rock;
- j=1..M, M =: the total number of measurements referred to under C);
- Wⱼ =: the weight attributed to the j-th measured conductivity of the rock;
- C_{t,j} =: the j-th calculated conductivity of the rock whose value depends on m* and n*;
- K_{t,j} =: the j-th measured conductivity of the rock;
- m* =: a parameter in the form of the cementation exponent, to be determined;
- n* =: a parameter in the form of the saturation exponent, to be determined.

The weights are chosen proportional to the inverse of the estimated accuracy variation of the measurement. The values of m* and n* are determined by minimising P(m*,n*) using a multi-dimensional down-hill-simplex method, which is an iterative mathematical method well-known to those skilled in the art.

The resulting values for m* and n* can then be used in the Waxman-Smits model to solve for S_{w} given the data referred to under B), thus providing the water-saturation (hence also the hydrocarbon saturation) in the earth formation.

## Claims

1. A method of determining a parameter of a physical model representing the electrical behaviour of a composition, the method comprising:
- defining said model by a relationship between at least one electrical property of the composition, a plurality of physical variables of the composition and said parameter;
- measuring a plurality of electrical properties including a first electrical property of the composition;
- selecting an incoherence function defining a difference between the measured electrical properties and the electrical properties as determined from said relationship, said incoherence function being such that the measured values are weighted in dependence of their accuracy; and
- determining said parameter by minimisation of said incoherence function, characterized in that said plurality of electrical properties includes a second electrical property of a sample which is representative for said composition, and that the method further comprises selecting the sample and measuring the second electrical property of the sample for various magnitudes of at least one of said physical variables.

2. The method of claim 1, wherein the step of determining said parameter by minimisation of said incoherence function includes an iterative process.

3. The method of claim 1 or 2, wherein a plurality of said parameters are determined simultaneously by minimising the incoherence function, each parameter being represented in said relationship.

4. The method of any of claims 1-3, wherein said incoherence function is selected such that independent measurements of substantially equal accuracy are substantially equally represented therein.

5. The method of any claims 1-4, wherein the at least one electrical property of the composition as defined in said relationship and the measured second electrical property of the sample are selected from the conductivity, the resistivity and the membrane potential.

6. The method of any of claims 1-5, wherein the measured first electrical property of the composition is selected from the conductivity and the resistivity.

7. The method of any of claims 1-6, wherein said composition includes a fluid and the model forms a saturation model for the fluid in the composition.

8. The method of claim 7, wherein said composition comprises an earth formation, and the model forms a saturation model for a selected one of brine and hydrocarbon fluid contained in the formation.

9. The method of any of claims 1-8, wherein said relationship is selected to include the electrical conductivity of the composition and a plurality of composition parameters including, for each component in the composition, physical parameters representing the electrical conductivity and the volume fraction of the component, said relationship being such that the components are substantially equally represented in said relationship by means of said physical parameters.

10. The method of claim 9, wherein the relationship is selected as:${\text{(σ}}_{\text{eff}} {\text{- σ}}_{\text{0}} {\text{), (Lσ}}_{\text{eff}} {\text{+ (1-L)σ}}_{\text{0}} {\text{)}}^{\text{-1}} {\text{= Σ φ}}_{\text{k}} {\text{(σ}}_{\text{k}} {\text{- σ}}_{\text{0}} {\text{).(Lσ}}_{\text{k}} {\text{+ (1-L)σ}}_{\text{0}} {\text{)}}^{\text{-1}}$ wherein
σ₀ represents an auxiliary parameter in the form of a conductivity tensor
k = 1 ... N, N being the number components in the composition
σ_{eff} represents the conductivity tensor of the sample σₖ represents the conductivity tensor of component k
φₖ represents the volume fraction of component k
L represents a depolarisation tensor.

11. The method of any of claims 1-8, wherein said model forms one of the Waxman-Smits model, the Archie model, the Poupon-Leveaux model, the Simandoux model, the Dual-Water model of Clavier-Coates-Dumanoir, and the effective medium model of Spalburg.

12. The method of any of claims 1-11, wherein the step of determining said parameter by minimisation of said incoherence function is carried out in a minimisation algorithm.

13. The method of any of claims 1-12, wherein said incoherence function is monotonically increasing with the deviation between the measured electrical properties and the calculated electrical properties.

14. The method of any of claims 1-13, wherein said incoherence function is of the form: 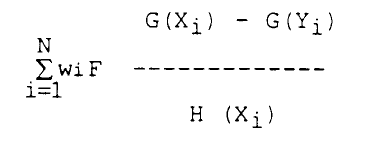 wherein
i=1..N, N being the number of physical measurements,
Xᵢ represents the measured electrical property,
Yᵢ represents the calculated electrical property,
G and H represent functions of the electrical property (H(Xᵢ)>0)
F represents a function with ∀*X*≠0[*F*(*X*)>*F*(0)],
wᵢ represents the weight attributed to the physical measurement (wᵢ≥0).

15. The method of claim 14, wherein${\text{F(X) = |X|}}^{\text{α}} \text{,α ∈ R, α > 0}$${\text{G(X) = X}}^{\text{β}} \text{,β ∈ R, β ≠ 0}$$\text{H(X) = 1 or H(X)= G (X).}$

16. The method of any of claims 1-13 wherein said incoherence function is of the form: 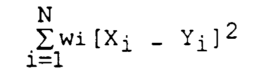 wherein
i=1..N, N being the number of physical measurements,
Xᵢ represents the measured electrical property,
Yᵢ represents the calculated electrical property,
wᵢ represents the weight attributed to the physical measurement (wᵢ≥0).

17. The method of any of claims 1-13, wherein said incoherence function is of the form: 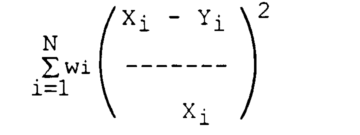 wherein
i=1..N, N being the number of physical measurements,
Xᵢ represents the measured electrical property,
Yᵢ represents the calculated electrical property,
wᵢ represents the weight attributed to the physical measurement (wᵢ≥0).

## Patentansprüche

1. Verfahren zum Bestimmen eines Parameters eines physikalischen Modells, welches das elektrische Verhalten einer Zusammensetzung darstellt, wobei das Verfahren umfaßt:
- Definieren des genannten Modells durch eine Beziehung zwischen zumindest einer elektrischen Eigenschaft der Zusammensetzung, einer Mehrzahl physikalischer Variablen der Zusammensetzung und dem genannten Parameter;
- Messen einer Mehrzahl von elektrischen Eigenschaften einschließlich einer ersten elektrischen Eigenschaft der Zusammensetzung;
- Auswählen einer Inkohärenzfunktion, welche eine Differenz zwischen den gemessenen elektrischen Eigenschaften und den aus der genannten Beziehung bestimmten elektrischen Eigenschaften definiert, welche Inkohärenzfunktion so ist, daß die gemessenen Werte in Abhängigkeit von ihrer Genauigkeit gewichtet sind; und
- Bestimmen des genannten Parameters durch Minimieren der Inkohärenzfunktion, dadurch gekennzeichnet, daß die genannte Mehrzahl von elektrischen Eigenschaften eine zweite elektrische Eigenschaft einer Probe einschließt, die für die genannte Zusammensetzung repräsentativ ist, und daß das Verfahren ferner das Auswählen der Probe und das Messen der zweiten elektrischen Eigenschaft der Probe bei verschiedenen Werten von zumindest einer der genannten physikalischen Variablen umfaßt.

2. Verfahren nach Anspruch 1, bei welchem der Schritt des Bestimmens des genannten Parameters durch Minimieren der genannten Inkohärenzfunktion einen iterativen Prozeß inkludiert.

3. Verfahren nach Anspruch 1 oder 2, bei welchem eine Vielzahl der genannten Parameter gleichzeitig bestimmt werden, indem die Inkohärenzfunktion minimiert wird, wobei jeder Parameter in der genannten Beziehung repräsentiert ist.

4. Verfahren nach einem der Ansprüche 1 bis 3, bei welchem die genannte Inkohärenzfunktion so gewählt ist, daß unabhängige Messungen von im wesentlichen gleicher Genauigkeit darin im wesentlichen gleich repräsentiert sind.

5. Verfahren nach einem der Ansrüche 1 bis 4, bei welchem die zumindest eine elektrische Eigenschaft der Zusammensetzung, wie sie in der genannten Beziehung definiert ist, und die gemessene zweite elektrische Eigenschaft der Probe aus der Leitfähigkeit, dem spezifischen Widerstand und dem Membranpotential gewählt sind.

6. Verfahren nach einem der Ansprüche 1 bis 5, bei welchem die gemessene erste elektrische Eigenschaft der Zusammensetzung aus der Leitfähigkeit und dem spezifischen Widerstand ausgewählt ist.

7. Verfahren nach einem der Ansprüche 1 bis 6, bei welchem die genannte Zusammensetzung ein Fluid enthält und das Modell ein Sättigungsmodell für das Fluid in der Zusammensetzung bildet.

8. Verfahren nach Anspruch 7, bei welchem die Zusammensetzung eine Erdformation umfaßt und das Modell ein Sättigungsmodell für eine ausgewählte Komponente der Komponenten Sole und Kohlenwasserstofffluid bildet, die in der Formation enthalten sind.

9. Verfahren nach einem der Ansprüche 1 bis 8, bei welchem die genannte Beziehung so gewählt ist, daß sie die elektrische Leitfähigkeit der Zusammensetzung und eine Vielzahl von Zusammensetzungsparametern enthält, darunter - für jede Komponente in der Zusammensetzung - die elektrische Leitfähigkeit und den Volumsanteil der Komponente repräsentierende physikalische Parameter, wobei die genannte Beziehung so ist, daß die Komponenten in der Beziehung mit Hilfe der genannten physikalischen Parameter im wesentlichen gleich repräsentiert sind.

10. Verfahren nach Anspruch 9, bei welchem die Beziehung gewählt wird zu:${\text{(σ}}_{\text{eff}} {\text{-σ}}_{\text{0}} {\text{) . (Lσ}}_{\text{eff}} {\text{+ (1-L)σ}}_{\text{0}} {\text{)}}^{\text{-1}} {\text{= Σφ}}_{\text{k}} {\text{(σ}}_{\text{k}} {\text{- σ}}_{\text{0}} {\text{). (Lσ}}_{\text{k}} {\text{+ (1-L)σ}}_{\text{0}} {\text{)}}^{\text{-1}}$ wobei
σ₀ einen Hilfsparameter in Form eines Leitfähigkeittensors darstellt;
k = 1 ... N, wobei N die Anzahl von Komponenten in der Zusammensetzung ist;
σ_{eff} den Leitfähigkeitstensor der Probe darstellt;
σₖ den Leitfähigkeitstensor der Komponente k darstellt;
φₖ den Volumsanteil der Komponente k darstellt; und
L einen Depolarisationstensor darstellt.

11. Verfahren nach einem der Ansprüche 1 bis 8, bei welchem das Modell eines der Modelle Waxman-Smits-Modell, Archie-Modell, Poupon-Leveaux-Modell, Simandoux-Modell, Dual Water-Modell von Clavier-Coates-Dumanoir und effektives-Medium-Modell von Spalburg bildet.

12. Verfahren nach einem der Ansprüche 1 bis 11, bei welchem der Schritt des Bestimmens des genannten Parameters durch Minimieren der genannten Inkohärenzfunktion in einem Minimierungsalgorithmus ausgeführt wird.

13. Verfahren nach einem der Ansprüche 1 bis 12, bei welchem die genannte Inkohärenzfunktion mit der Abweichung zwischen den gemessenen elektrischen Eigenschaften und den berechneten elektrischen Eigenschaften monoton ansteigend ist.

14. Verfahren nach einem der Ansprüche 1 bis 13, bei welchem die genannte Inkohärenzfunktion in der Form: 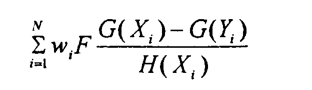 ist, wobei
i=1..N, wobei N die Anzahl der physikalischen Messungen ist,
Xᵢ die gemessene elektrische Eigenschaft darstellt,
Y₁ die berechnete elektrische Eigenschaft darstellt,
G und H Funktionen der elektrischen Eigenschaft darstellen (H(Xᵢ)>0),
F eine Funktion mit ∀*X*≠0[*F*(*X*)>*F*(0)] darstellt, und
wᵢ das der physikalischen Messung zugeordnete Gewicht darstellt (wᵢ≥0).

15. Verfahren nach Anspruch 14, bei welchem${\text{F(X) = |X|}}^{\text{α}} \text{, α ∈ R, α > 0}$${\text{G(X) = X}}^{\text{β}} \text{, β ∈ R, β ≠ 0}$$\text{H(X) = 1 oder H(X) = G(X).}$

16. Verfahren nach einem der Ansprüche 1 bis 13, bei welchem die Inkohärenzfunktion in der Form: 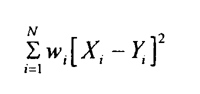 ist, wobei
i=1..N, wobei N die Anzahl der physikalischen Messungen ist,
Xᵢ die gemessene elektrische Eigenschaft darstellt,
Yᵢ die berechnete elektrische Eigenschaft darstellt, und
wᵢ das der physikalischen Messung zugeordnete Gewicht darstellt (wᵢ≥0).

17. Verfahren nach einem der Ansprüche 1 bis 13, bei welchem die Inkohärenzfunktion in der Form: 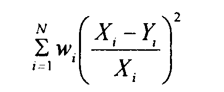 ist, wobei
i=1..N, wobei N die Anzahl der physikalischen Messungen ist,
Xᵢ die gemessene elektrische Eigenschaft darstellt,
Yᵢ die berechnete elektrische Eigenschaft darstellt, und
wᵢ das der physikalischen Messung zugeordnete Gewicht darstellt (wᵢ≥0).

## Revendications

1. Procédé de détermination d'un paramètre d'un modèle physique représentant le comportement électrique d'une composition, lequel procédé comprenant :
- la définition dudit modèle par une relation entre au moins une propriété électrique de la composition, une pluralité de variables physiques de la composition et ledit paramètre;
- la mesure d'une pluralité de propriétés électriques comprenant une première propriété électrique de la composition;
- la sélection d'une fonction d'incohérence définissant une différence entre les propriétés électriques mesurées et les propriétés électriques telles que déterminées à partir de ladite relation, ladite fonction d'incohérence étant telle que les valeurs mesurées sont pondérées en dépendance de leur précision; et
- la détermination du paramètre susdit par une minimisation de la fonction d'incohérence susdite, caractérisé en ce que ladite pluralité de propriétés électriques comprend une seconde propriété électrique d'un échantillon qui est représentative de la composition précitée, et en ce que le procédé comprend de plus la sélection de l'échantillon et la mesure de la seconde propriété électrique de l'échantillon pour diverses grandeurs d'au moins une des variables physiques précitées.

2. Procédé suivant la revendication 1, dans lequel l'étape de détermination du paramètre précité par une minimisation de la fonction d'incohérence précitée comprend un procédé itératif.

3. Procédé suivant l'une ou l'autre des revendications 1 et 2, dans lequel une pluralité desdits paramètres sont déterminés simultanément en minimisant la fonction d'incohérence, chaque paramètre étant représenté dans la relation précitée.

4. Procédé suivant l'une quelconque des revendications 1 à 3, dans lequel la fonction d'incohérence est choisie de telle sorte que des mesures indépendantes de précision sensiblement égale y soient sensiblement également représentées.

5. Procédé suivant l'une quelconque des revendications 1 à 4, dans lequel la au moins une propriété électrique de la composition telle que définie dans la relation précitée et la seconde propriété électrique mesurée de l'échantillon sont choisies parmi la conductivité, la résistivité et le potentiel de membrane.

6. Procédé suivant l'une quelconque des revendications 1 à 5, dans lequel la première propriété électrique mesurée de la composition est choisie parmi la conductivité et la résistivité.

7. Procédé suivant l'une quelconque des revendications 1 à 6, dans lequel ladite composition comprend un fluide et le modèle forme un modèle de saturation pour le fluide dans la composition.

8. Procédé suivant la revendication 7, dans lequel ladite composition comprend une formation terrestre et le modèle forme un modèle de saturation pour un fluide choisi parmi la saumure et un fluide hydrocarboné contenus dans la formation.

9. Procédé suivant l'une quelconque des revendications 1 à 8, dans lequel la relation précitée est choisie pour inclure la conductivité électrique de la composition et une pluralité de paramètres de la composition comprenant, pour chaque composant dans la composition, des paramètres physiques représentant la conductivité électrique et la fraction volumique du composant, ladite relation étant telle que les composants sont sensiblement également représentés dans la relation au moyen desdits paramètres physiques.

10. Procédé suivant la revendication 9, dans lequel la relation est choisie comme étant :${\text{(σ}}_{\text{eff}} {\text{- σ}}_{\text{0}} {\text{). (Lσ}}_{\text{eff}} {\text{+ (1-L)σ}}_{\text{0}} {\text{)}}^{\text{-1}} {\text{= Σ φ}}_{\text{k}} {\text{(σ}}_{\text{k}} {\text{- σ}}_{\text{0}} {\text{).(Lσ}}_{\text{k}} {\text{+ (1-L)σ}}_{\text{0}} {\text{)}}^{\text{-1}}$ dans laquelle
σ₀ représente un paramètre auxiliaire sous la forme d'un tenseur de conductivité,
k=1..N, N étant le nombre de composants dans la composition,
σ_{eff} représente le tenseur de conductivité de l'échantillon,
σₖ représente le tenseur de conductivité du composant k,
φₖ représente la fraction volumique du composant k,
L représente un tenseur de dépolarisation.

11. Procédé suivant l'une quelconque des revendications 1 à 8, dans lequel le modèle précité est un modèle parmi le modèle de Waxman-Smits, le modèle d'Archie, le modèle de Poupon-Leveaux, le modèle de Simandoux, le modèle à Eau Double de Clavier-Coates-Dumanoir et le modèle de milieu effectif de Spalburg.

12. Procédé suivant l'une quelconque des revendications 1 à 11, dans lequel l'étape de détermination du paramètre précité par une minimisation de la fonction d'incohérence précitée est réalisée dans un algorithme de minimisation.

13. Procédé suivant l'une quelconque des revendications 1 à 12, dans lequel ladite fonction d'incohérence est élevée monotoniquement avec l'écart entre les propriétés électriques mesurées et les propriétés électriques calculées.

14. Procédé suivant l'une quelconque des revendications 1 à 13, dans lequel la fonction d'incohérence est sous la forme : 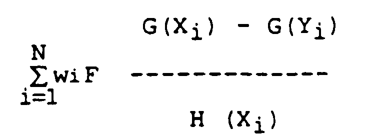 dans laquelle :
i=1..N, N étant le nombre de mesures physiques,
Xᵢ représente la propriété électrique mesurée,
Yᵢ représente la propriété électrique calculée,
G et H représentent des fonctions de la propriété électrique (H(Xᵢ)>0),
F représente une fonction avec ∀*X*≠0[*F*(*X*)>*F*(0)],
Wᵢ représente la pondération attribuée à la mesure physique (wᵢ≥0).

15. Procédé suivant la revendication 14, dans lequel${\text{F(X) = |X|}}^{\text{α}} \text{,α ∈ R, α > 0}$${\text{G(X) = X}}^{\text{β}} \text{,β ∈ R, β≠0}$$\text{H(X) = 1 ou H(X)=G (X).}$

16. Procédé suivant l'une quelconque des revendications 1 à 13, dans lequel la fonction d'incohérence est sous la forme : 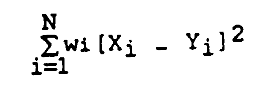 dans laquelle :
i=1..N, N étant le nombre de mesures physiques,
Xᵢ représente la propriété électrique mesurée,
Yᵢ représente la propriété électrique calculée,
Wᵢ représente la pondération attribuée à la mesure physique (wᵢ≥0).

17. Procédé suivant l'une quelconque des revendications 1 à 13, dans lequel la fonction d'incohérence est sous la forme : 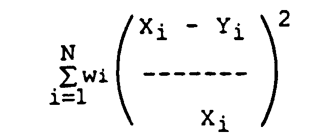 dans laquelle :
i=1..N, N étant le nombre de mesures physiques,
Xᵢ représente la propriété électrique mesurée,
Yᵢ représente la propriété électrique calculée,
Wᵢ représente la pondération attribuée à la mesure physique (wᵢ≥0).
